# EUROPEAN PATENT SPECIFICATION

(11) **EP 3 711 700 B1**
(45) Date of publication and mention of the grant of the patent: **06.12.2023**
(21) Application number: 20163554.7
(22) Date of filing: 17.03.2020
(51) Int. Cl.: A61B 34/00, A61B 90/00, A61B 34/10, A61B 34/20, A61B 34/30, A61B 90/10, A61B 90/14

(54) **SYSTEM FOR NEURONAVIGATION REGISTRATION AND ROBOTIC TRAJECTORY GUIDANCE, AND RELATED METHODS AND DEVICES**
SYSTEM ZUR REGISTRIERUNG VON NEURONAVIGATION UND ROBOTERBAHNFÜHRUNG SOWIE DAMIT VERBUNDENE VERFAHREN UND VORRICHTUNGEN
SYSTÈME D'ENREGISTREMENT DE NEURONAVIGATION ET DE GUIDAGE ROBOTIQUE DE TRAJECTOIRE ET PROCÉDÉS ET DISPOSITIFS ASSOCIÉS

(30) Priority: 22.03.2019 US 201916361863
(43) Date of publication of application: 23.09.2020
(73) Proprietor: Globus Medical, Inc., Audubon, PA 19403 (US)
(72) Inventor: CAMERON, Hayden, Philadelphia, PA 19148 (US); MANTZAVINOS, Spiros, Nashua, MA 03064 (US); CRAWFORD, Neil R., Chandler, AZ 85224 (US); JOSHI, Sanjay M., Andover, MA 01810 (US); JOHNSON, Norbert, North Andover, MA 01845 (US)
(74) Representative: Morabito, Sara

(56) References cited:
- US-A1- 2018 325 610
- US-A1- 2019 029 765

## Description

### FIELD

The present disclosure relates to medical devices and systems, and more particularly, systems for neuronavigation registration and robotic trajectory guidance, and related methods and devices.

### BACKGROUND

Position recognition systems for robot assisted surgeries are used to determine the position of and track a particular object in 3-dimensions (3D). In robot assisted surgeries, for example, certain objects, such as surgical instruments, need to be tracked with a high degree of precision as the instrument is being positioned and moved by a robot or by a physician, for example.

Position recognition systems may use passive and/or active sensors or markers for registering and tracking the positions of the objects. Using these sensors, the system may geometrically resolve the 3-dimensional position of the sensors based on information from or with respect to one or more cameras, signals, or sensors, etc. These surgical systems can therefore utilize position feedback to precisely guide movement of robotic arms and tools relative to a patients' surgical site. Thus, there is a need for a system that efficiently and accurately provide neuronavigation registration and robotic trajectory guidance in a surgical environment.

US2018/325610 and US2019/029765 describe a navigation system known in the art.

### SUMMARY

According to the invention it is provided a navigation system having the features of the independent claim 1. Further advantageous versions of the system of the invention are set forth in the dependent claims.

According to some embodiments of inventive concepts, a system includes a processor circuit and a memory coupled to the processor circuit. The memory includes machine-readable instructions configured to cause the processor circuit to determine, based on a first image volume comprising an anatomical feature of a patient, a registration fixture that is fixed with respect to the anatomical feature of the patient, and a first plurality of fiducial markers that are fixed with respect to the registration fixture, determine, for each fiducial marker of the first plurality of fiducial markers, a position of the fiducial marker relative to the image volume. The machine-readable instructions are further configured to cause the processor circuit to determine, based on the determined positions of the first plurality of fiducial markers, a position and orientation of the registration fixture with respect to the anatomical feature. The machine-readable instructions are further configured to cause the processor circuit to, based on a data frame from a tracking system comprising a second plurality of tracking markers that are fixed with respect to the registration fixture, determine, for each tracking marker of the second plurality of tracking markers, a position of the tracking marker. The machine-readable instructions are further configured to cause the processor circuit to determine, based on the determined positions of the second plurality of tracking markers, a position and orientation of the registration fixture with respect to a robot arm of a surgical robot. The machine-readable instructions are further configured to cause the processor circuit to determine, based on the determined position and orientation of the registration fixture with respect to the anatomical feature and the determined position and orientation of the registration fixture with respect to the robot arm, a position and orientation of the anatomical feature with respect to the robot arm. The machine-readable instructions are further configured to cause the processor circuit to control the robot arm based on the determined position and orientation of the anatomical feature with respect to the robot arm.

According to some other embodiments of inventive concepts, a computer-implemented method is disclosed. The computer-implemented method includes, based on a first image volume comprising an anatomical feature of a patient, a registration fixture that is fixed with respect to the anatomical feature of the patient, and a first plurality of fiducial markers that are fixed with respect to the registration fixture, determining, for each fiducial marker of the first plurality of fiducial markers, a position of the fiducial marker. The computer-implemented method further includes determining, based on the determined positions of the first plurality of fiducial markers, a position and orientation of the registration fixture with respect to the anatomical feature. The computer-implemented method further includes, based on a tracking data frame comprising a second plurality of tracking markers that are fixed with respect to the registration fixture, determining, for each tracking marker of the second plurality of tracking markers, a position of the tracking marker. The computer-implemented method further includes determining, based on the determined positions of the second plurality of tracking markers, a position and orientation of the registration fixture with respect to a robot arm of a surgical robot. The computer-implemented method further includes determining, based on the determined position and orientation of the registration fixture with respect to the anatomical feature and the determined position and orientation of the registration fixture with respect to the robot arm, a position and orientation of the anatomical feature with respect to the robot arm. The computer-implemented method further includes controlling the robot arm based on the determined position and orientation of the anatomical feature with respect to the robot arm.

According to some other embodiments of inventive concepts, a surgical system is disclosed. The surgical system includes an intraoperative surgical tracking computer having a processor circuit and a memory. The memory includes machine-readable instructions configured to cause the processor circuit to provide a medical image volume defining an image space. The medical image volume includes an anatomical feature of a patient, a registration fixture that is fixed with respect to the anatomical feature of the patient, and a plurality of fiducial markers that are fixed with respect to the registration fixture. The machine-readable instructions are further configured to cause the processor circuit to, based on the medical image volume, determine, for each fiducial marker of the plurality of fiducial markers, a position of the fiducial marker with respect to the image space. The machine-readable instructions are further configured to cause the processor circuit to determine, based on the determined positions of the plurality of fiducial markers, a position and orientation of the registration fixture with respect to the anatomical feature. The machine-readable instructions are further configured to cause the processor circuit to provide a tracking data frame defining a tracking space, the tracking data frame comprising positions of a first plurality of tracked markers that are fixed with respect to the registration fixture. The machine-readable instructions are further configured to cause the processor circuit to, based on the tracking data frame, determine a position of the anatomical feature with respect to the first plurality of tracked markers in the tracking space. The surgical system further includes a surgical robot having a robot arm configured to position a surgical end-effector. The surgical robot further includes a controller connected to the robot arm. The controller is configured to perform operations including, based on the tracking data frame, determining a position of the robot arm with respect to the tracking space. The controller is configured to perform operations including determining, based on the determined position and orientation of the anatomical feature with respect to the tracking space and the determined position and orientation of the robot arm with respect to the tracking space, a position and orientation of the anatomical feature with respect to the robot arm. The controller is configured to perform operations including controlling movement of the robot arm based on the determined position and orientation of the anatomical feature with respect to the robot arm to position the surgical end-effector relative to a location on the patient to facilitate surgery on the patient.

Other methods and related devices and systems, and corresponding methods and computer program products according to embodiments will be or become apparent to one with skill in the art upon review of the following drawings and detailed description. It is intended that all such devices and systems, and corresponding methods and computer program products be included within this description, be within the scope of the present disclosure, and be protected by the accompanying claims. Moreover, it is intended that all embodiments disclosed herein can be implemented separately or combined in any way and/or combination.

### BRIEF DESCRIPTION OF THE DRAWINGS

The accompanying drawings, which are included to provide a further understanding of the disclosure and are incorporated in a constitute a part of this application, illustrate certain non-limiting embodiments of inventive concepts. In the drawings:
FIG. 1A is an overhead view of an arrangement for locations of a robotic system, patient, surgeon, and other medical personnel during a surgical procedure, according to some embodiments;
FIG. 1B is an overhead view of an alternate arrangement for locations of a robotic system, patient, surgeon, and other medical personnel during a cranial surgical procedure, according to some embodiments;
FIG. 2 illustrates a robotic system including positioning of the surgical robot and a camera relative to the patient according to some embodiments;
FIG. 3 is a flowchart diagram illustrating computer-implemented operations for determining a position and orientation of an anatomical feature of a patient with respect to a robot arm of a surgical robot, according to some embodiments;
FIG. 4 is a diagram illustrating processing of data for determining a position and orientation of an anatomical feature of a patient with respect to a robot arm of a surgical robot, according to some embodiments;
FIGS. 5A-5C illustrate a system for registering an anatomical feature of a patient using a computerized tomography (CT) localizer, a frame reference array (FRA), and a dynamic reference base (DRB), according to some embodiments;
FIGS. 6A and 6B illustrate a system for registering an anatomical feature of a patient using fluoroscopy (fluoro) imaging, according to some embodiments;
FIG. 7 illustrates a system for registering an anatomical feature of a patient using an intraoperative CT fixture (ICT) and a DRB, according to some embodiments;
FIGS. 8A and 8B illustrate systems for registering an anatomical feature of a patient using a DRB and an X-ray cone beam imaging device, according to some embodiments;
FIG. 9 illustrates a system for registering an anatomical feature of a patient using a navigated probe and fiducials for point-to-point mapping of the anatomical feature, according to some embodiments;
FIG. 10 illustrates a two-dimensional visualization of an adjustment range for a centerpoint-arc mechanism, according to some embodiments; and
FIG. 11 illustrates a two-dimensional visualization of virtual point rotation mechanism, according to some embodiments.

### DETAILED DESCRIPTION

It is to be understood that the present disclosure is not limited in its application to the details of construction and the arrangement of components set forth in the description herein or illustrated in the drawings. The teachings of the present disclosure may be used and practiced in other embodiments and practiced or carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Unless specified or limited otherwise, the terms "mounted," "connected," "supported," and "coupled" and variations thereof are used broadly and encompass both direct and indirect mountings, connections, supports, and couplings. Further, "connected" and "coupled" are not restricted to physical or mechanical connections or couplings.

The following discussion is presented to enable a person skilled in the art to make and use embodiments of the present disclosure. Various modifications to the illustrated embodiments will be readily apparent to those skilled in the art, and the principles herein can be applied to other embodiments and applications without departing from embodiments of the present disclosure. Thus, the embodiments are not intended to be limited to embodiments shown, but are to be accorded the widest scope consistent with the principles and features disclosed herein. The following detailed description is to be read with reference to the figures, in which like elements in different figures have like reference numerals. The figures, which are not necessarily to scale, depict selected embodiments and are not intended to limit the scope of the embodiments. Skilled artisans will recognize the examples provided herein have many useful alternatives and fall within the scope of the embodiments.

According to some other embodiments, systems for neuronavigation registration and robotic trajectory guidance, and related methods and devices are disclosed. In some embodiments, a first image having an anatomical feature of a patient, a registration fixture that is fixed with respect to the anatomical feature of the patient, and a first plurality of fiducial markers that are fixed with respect to the registration fixture is analyzed, and a position is determined for each fiducial marker of the first plurality of fiducial markers. Next, based on the determined positions of the first plurality of fiducial markers, a position and orientation of the registration fixture with respect to the anatomical feature is determined. A data frame comprising a second plurality of tracking markers that are fixed with respect to the registration fixture is also analyzed, and a position is determined for each tracking marker of the second plurality of tracking markers. Based on the determined positions of the second plurality of tracking markers, a position and orientation of the registration fixture with respect to a robot arm of a surgical robot is determined. Based on the determined position and orientation of the registration fixture with respect to the anatomical feature and the determined position and orientation of the registration fixture with respect to the robot arm, a position and orientation of the anatomical feature with respect to the robot arm is determined, which allows the robot arm to be controlled based on the determined position and orientation of the anatomical feature with respect to the robot arm.

Advantages of this and other embodiments include the ability to combine neuronavigation and robotic trajectory alignment into one system, with support for a wide variety of different registration hardware and methods. For example, as will be described in detail below, embodiments may support both computerized tomography (CT) and fluoroscopy (fluoro) registration techniques, and may utilize frame-based and/or frameless surgical arrangements. Moreover, in many embodiments, if an initial (e.g. preoperative) registration is compromised due to movement of a registration fixture, registration of the registration fixture (and of the anatomical feature by extension) can be re-established intraoperatively without suspending surgery and re-capturing preoperative images.

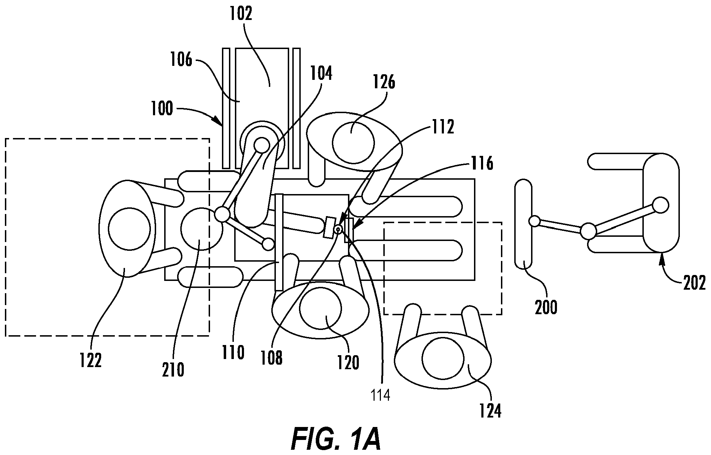

Referring now to the drawings, FIG. 1A illustrates a surgical robot system 100 in accordance with an embodiment. Surgical robot system 100 may include, for example, a surgical robot 102, one or more robot arms 104, a base 106, a display 110, an end-effector 112, for example, including a guide tube 114, and one or more tracking markers 118. The robot arm 104 may be movable along and/or about an axis relative to the base 106, responsive to input from a user, commands received from a processing device, or other methods. The surgical robot system 100 may include a patient tracking device 116 also including one or more tracking markers 118, which is adapted to be secured directly to the patient 210 (e.g., to a bone of the patient 210). As will be discussed in greater detail below, the tracking markers 118 may be secured to or may be part of a stereotactic frame that is fixed with respect to an anatomical feature of the patient 210. The stereotactic frame may also be secured to a fixture to prevent movement of the patient 210 during surgery.

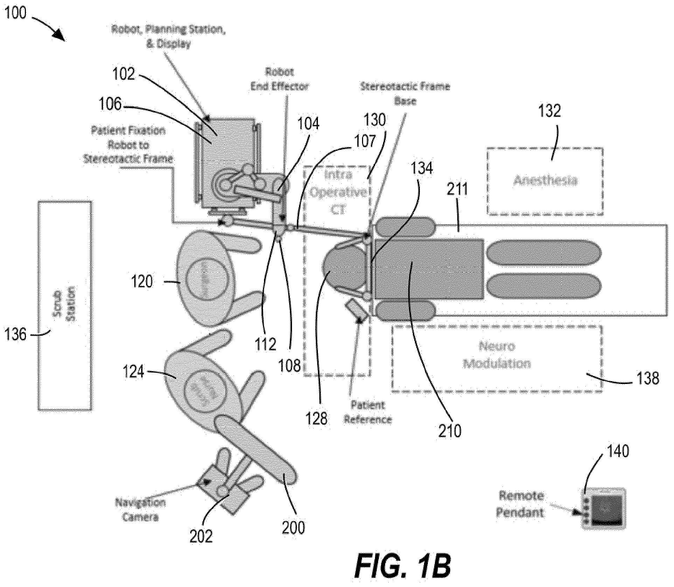

According to an alternative embodiment, FIG. 1B is an overhead view of an alternate arrangement for locations of a robotic system 100, patient 210, surgeon 120, and other medical personnel during a cranial surgical procedure. During a cranial procedure, for example, the robot 102 may be positioned behind the head 128 of the patient 210. The robot arm 104 of the robot 102 has an end-effector 112 that may hold a surgical instrument 108 during the procedure. In this example, a stereotactic frame 134 is fixed with respect to the patient's head 128, and the patient 210 and/or stereotactic frame 134 may also be secured to a patient base 211 to prevent movement of the patient's head 128 with respect to the patient base 211. In addition, the patient 210, the stereotactic frame 134 and/or or the patient base 211 may be secured to the robot base 106, such as via an auxiliary arm 107, to prevent relative movement of the patient 210 with respect to components of the robot 102 during surgery. Different devices may be positioned with respect to the patient's head 128 and/or patient base 211 as desired to facilitate the procedure, such as an intra-operative CT device 130, an anesthesiology station 132, a scrub station 136, a neuro-modulation station 138, and/or one or more remote pendants 140 for controlling the robot 102 and/or other devices or systems during the procedure.

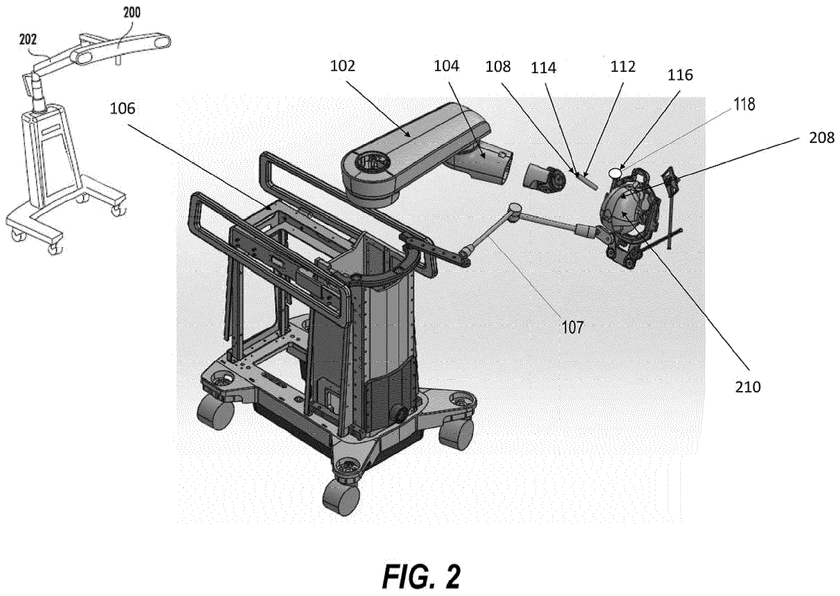

The surgical robot system 100 in the examples of FIGS. 1A and/or 1B may also use a sensor, such as a camera 200, for example, positioned on a camera stand 202. The camera stand 202 can have any suitable configuration to move, orient, and support the camera 200 in a desired position. The camera 200 may include any suitable camera or cameras, such as one or more cameras (e.g., bifocal or stereophotogrammetric cameras), able to identify, for example, active or passive tracking markers 118 (shown as part of patient tracking device 116 in FIG. 2) in a given measurement volume viewable from the perspective of the camera 200. In this example, the camera 200 may scan the given measurement volume and detect the light that comes from the tracking markers 118 in order to identify and determine the position of the tracking markers 118 in three-dimensions. For example, active tracking markers 118 may include infrared-emitting markers that are activated by an electrical signal (e.g., infrared light emitting diodes (LEDs)), and/or passive tracking markers 118 may include retro-reflective markers that reflect infrared or other light (e.g., they reflect incoming IR radiation into the direction of the incoming light), for example, emitted by illuminators on the camera 200 or other suitable sensor or other device.

In many surgical procedures, one or more targets of surgical interest, such as targets within the brain for example, are localized to an external reference frame. For example, stereotactic neurosurgery may use an externally mounted stereotactic frame that facilitates patient localization and implant insertion via a frame mounted arc. Neuronavigation is used to register, e.g., map, targets within the brain based on pre-operative or intraoperative imaging. Using this pre-operative or intraoperative imaging, links and associations can be made between the imaging and the actual anatomical structures in a surgical environment, and these links and associations can be utilized by robotic trajectory systems during surgery.

According to some embodiments, various software and hardware elements may be combined to create a system that can be used to plan, register, place and verify the location of an instrument or implant in the brain. These systems may integrate a surgical robot, such as the surgical robot 102 of FIGS. 1A and/or 1B, and may employ a surgical navigation system and planning software to program and control the surgical robot. In addition or alternatively, the surgical robot 102 may be remotely controlled, such as by nonsterile personnel.

The robot 102 may be positioned near or next to patient 210, and it will be appreciated that the robot 102 can be positioned at any suitable location near the patient 210 depending on the area of the patient 210 undergoing the operation. The camera 200 may be separated from the surgical robot system 100 and positioned near or next to patient 210 as well, in any suitable position that allows the camera 200 to have a direct visual line of sight to the surgical field 208. In the configuration shown, the surgeon 120 may be positioned across from the robot 102, but is still able to manipulate the end-effector 112 and the display 110. A surgical assistant 126 may be positioned across from the surgeon 120 again with access to both the end-effector 112 and the display 110. If desired, the locations of the surgeon 120 and the assistant 126 may be reversed. The traditional areas for the anesthesiologist 122 and the nurse or scrub tech 124 may remain unimpeded by the locations of the robot 102 and camera 200.

With respect to the other components of the robot 102, the display 110 can be attached to the surgical robot 102 and in other embodiments, the display 110 can be detached from surgical robot 102, either within a surgical room with the surgical robot 102, or in a remote location. The end-effector 112 may be coupled to the robot arm 104 and controlled by at least one motor. In some embodiments, end-effector 112 can comprise a guide tube 114, which is able to receive and orient a surgical instrument 108 used to perform surgery on the patient 210. As used herein, the term "end-effector" is used interchangeably with the terms "end-effectuator" and "effectuator element." Although generally shown with a guide tube 114, it will be appreciated that the end-effector 112 may be replaced with any suitable instrumentation suitable for use in surgery. In some embodiments, end-effector 112 can comprise any known structure for effecting the movement of the surgical instrument 108 in a desired manner.

The surgical robot 102 is able to control the translation and orientation of the end-effector 112. The robot 102 is able to move end-effector 112 along x-, y-, and z-axes, for example. The end-effector 112 can be configured for selective rotation about one or more of the x-, y-, and z-axis such that one or more of the Euler Angles (e.g., roll, pitch, and/or yaw) associated with end-effector 112 can be selectively controlled. In some embodiments, selective control of the translation and orientation of end-effector 112 can permit performance of medical procedures with significantly improved accuracy compared to conventional robots that use, for example, a six degree of freedom robot arm comprising only rotational axes. For example, the surgical robot system 100 may be used to operate on patient 210, and robot arm 104 can be positioned above the body of patient 210, with end-effector 112 selectively angled relative to the z-axis toward the body of patient 210.

In some embodiments, the position of the surgical instrument 108 can be dynamically updated so that surgical robot 102 can be aware of the location of the surgical instrument 108 at all times during the procedure. Consequently, in some embodiments, surgical robot 102 can move the surgical instrument 108 to the desired position quickly without any further assistance from a physician (unless the physician so desires). In some further embodiments, surgical robot 102 can be configured to correct the path of the surgical instrument 108 if the surgical instrument 108 strays from the selected, preplanned trajectory. In some embodiments, surgical robot 102 can be configured to permit stoppage, modification, and/or manual control of the movement of end-effector 112 and/or the surgical instrument 108. Thus, in use, in some embodiments, a physician or other user can operate the system 100, and has the option to stop, modify, or manually control the autonomous movement of end-effector 112 and/or the surgical instrument 108. Further details of surgical robot system 100 including the control and movement of a surgical instrument 108 by surgical robot 102 can be found in co-pending U.S. Patent Publication No. 2013/0345718, to which reference is made for that part.

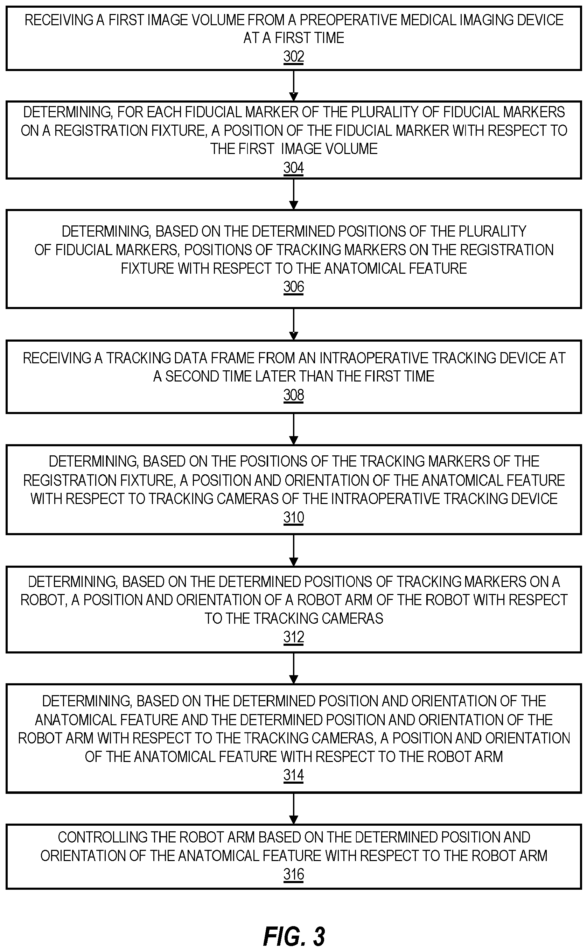

As will be described in greater detail below, the surgical robot system 100 can comprise one or more tracking markers configured to track the movement of robot arm 104, end-effector 112, patient 210, and/or the surgical instrument 108 in three dimensions. In some embodiments, a plurality of tracking markers can be mounted (or otherwise secured) thereon to an outer surface of the robot 102, such as, for example and without limitation, on base 106 of robot 102, on robot arm 104, and/or on the end-effector 112. In some embodiments, such as the embodiment of FIG. 3 below, for example, one or more tracking markers can be mounted or otherwise secured to the end-effector 112. One or more tracking markers can further be mounted (or otherwise secured) to the patient 210. In some embodiments, the plurality of tracking markers can be positioned on the patient 210 spaced apart from the surgical field 208 to reduce the likelihood of being obscured by the surgeon, surgical tools, or other parts of the robot 102. Further, one or more tracking markers can be further mounted (or otherwise secured) to the surgical instruments 108 (e.g., a screw driver, dilator, implant inserter, or the like). Thus, the tracking markers enable each of the marked objects (e.g., the end-effector 112, the patient 210, and the surgical instruments 108) to be tracked by the surgical robot system 100. In some embodiments, system 100 can use tracking information collected from each of the marked objects to calculate the orientation and location, for example, of the end-effector 112, the surgical instrument 108 (e.g., positioned in the tube 114 of the end-effector 112), and the relative position of the patient 210. Further details of surgical robot system 100 including the control, movement and tracking of surgical robot 102 and of a surgical instrument 108 can be found in U.S. Patent Publication No. 2016/0242849 to which reference is made for that part.

In some embodiments, pre-operative imaging may be used to identify the anatomy to be targeted in the procedure. If desired by the surgeon the planning package will allow for the definition of a reformatted coordinate system. This reformatted coordinate system will have coordinate axes anchored to specific anatomical landmarks, such as the anterior commissure (AC) and posterior commissure (PC) for neurosurgery procedures. In some embodiments, multiple pre-operative exam images (e.g., CT or magnetic resonance (MR) images) may be co-registered such that it is possible to transform coordinates of any given point on the anatomy to the corresponding point on all other pre-operative exam images.

As used herein, registration is the process of determining the coordinate transformations from one coordinate system to another. For example, in the co-registration of preoperative images, co-registering a CT scan to an MR scan means that it is possible to transform the coordinates of an anatomical point from the CT scan to the corresponding anatomical location in the MR scan. It may also be advantageous to register at least one exam image coordinate system to the coordinate system of a common registration fixture, such as a dynamic reference base (DRB), which may allow the camera 200 to keep track of the position of the patient in the camera space in real-time so that any intraoperative movement of an anatomical point on the patient in the room can be detected by the robot system 100 and accounted for by compensatory movement of the surgical robot 102.

FIG. 3 is a flowchart diagram illustrating computer-implemented operations 300 for determining a position and orientation of an anatomical feature of a patient with respect to a robot arm of a surgical robot, according to some embodiments. The operations 300 may include receiving a first image volume, such as a CT scan, from a preoperative image capture device at a first time (Block 302). The first image volume includes an anatomical feature of a patient and at least a portion of a registration fixture that is fixed with respect to the anatomical feature of the patient. The registration fixture includes a first plurality of fiducial markers that are fixed with respect to the registration fixture. The operations 300 further include determining, for each fiducial marker of the first plurality of fiducial markers, a position of the fiducial marker relative to the first image volume (Block 304). The operations 300 further include, determining, based on the determined positions of the first plurality of fiducial markers, positions of an array of tracking markers on the registration fixture (fiducial registration array or FRA) with respect to the anatomical feature (Block 306).

The operations 300 may further include receiving a tracking data frame from an intraoperative tracking device comprising a plurality of tracking cameras at a second time that is later than the first time (Block 308). The tracking frame includes positions of a plurality of tracking markers that are fixed with respect to the registration fixture (FRA) and a plurality of tracking markers that are fixed with respect to the robot. The operations 300 further include determining, for based on the positions of tracking markers of the registration fixture, a position and orientation of the anatomical feature with respect to the tracking cameras (Block 310). The operations 300 further include determining, based on the determined positions of the plurality of tracking markers on the robot, a position and orientation of the robot arm of a surgical robot with respect to the tracking cameras (Block 312).

The operations 300 further include determining, based on the determined position and orientation of the anatomical feature with respect to the tracking cameras and the determined position and orientation of the robot arm with respect to the tracking cameras, a position and orientation of the anatomical feature with respect to the robot arm (Block 314). The operations 300 further include controlling movement of the robot arm with respect to the anatomical feature, e.g., along and/or rotationally about one or more defined axis, based on the determined position and orientation of the anatomical feature with respect to the robot arm (Block 316).

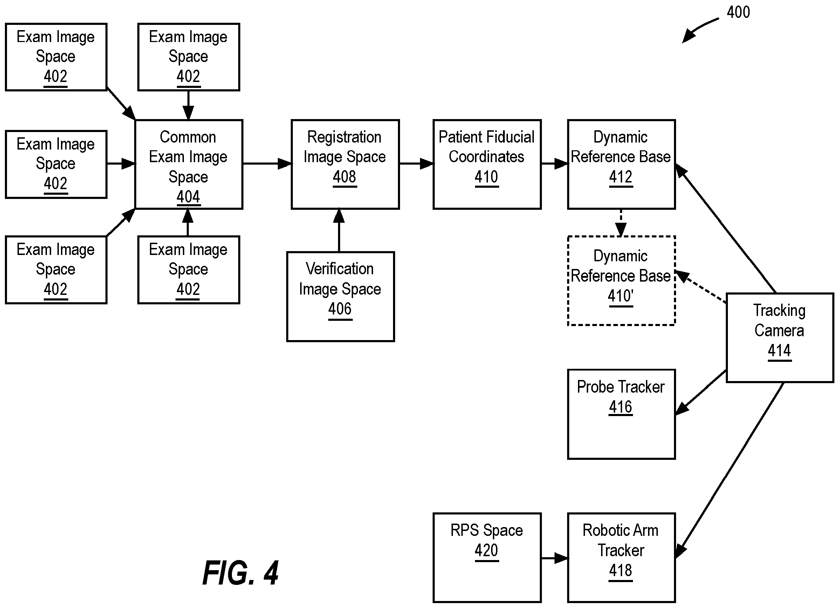

FIG. 4 is a diagram illustrating a data flow 400 for a multiple coordinate transformation system, to enable determining a position and orientation of an anatomical feature of a patient with respect to a robot arm of a surgical robot, according to some embodiments. In this example, data from a plurality of exam image spaces 402, based on a plurality of exam images, may be transformed and combined into a common exam image space 404. The data from the common exam image space 404 and data from a verification image space 406, based on a verification image, may be transformed and combined into a registration image space 408. Data from the registration image space 408 may be transformed into patient fiducial coordinates 410, which is transformed into coordinates for a DRB 412. A tracking camera 414 may detect movement of the DRB 412 (represented by DRB 412') and may also detect a location of a probe tracker 416 to track coordinates of the DRB 412 over time. A robotic arm tracker 418 determines coordinates for the robot arm based on transformation data from a Robotics Planning System (RPS) space 420 or similar modeling system, and/or transformation data from the tracking camera 414.

It should be understood that these and other features may be used and combined in different ways to achieve registration of image space, i.e., coordinates from image volume, into tracking space, i.e., coordinates for use by the surgical robot in real-time. As will be discussed in detail below, these features may include fiducial-based registration such as stereotactic frames with CT localizer, preoperative CT or MRI registered using intraoperative fluoroscopy, calibrated scanner registration where any acquired scan's coordinates are pre-calibrated relative to the tracking space, and/or surface registration using a tracked probe, for example.

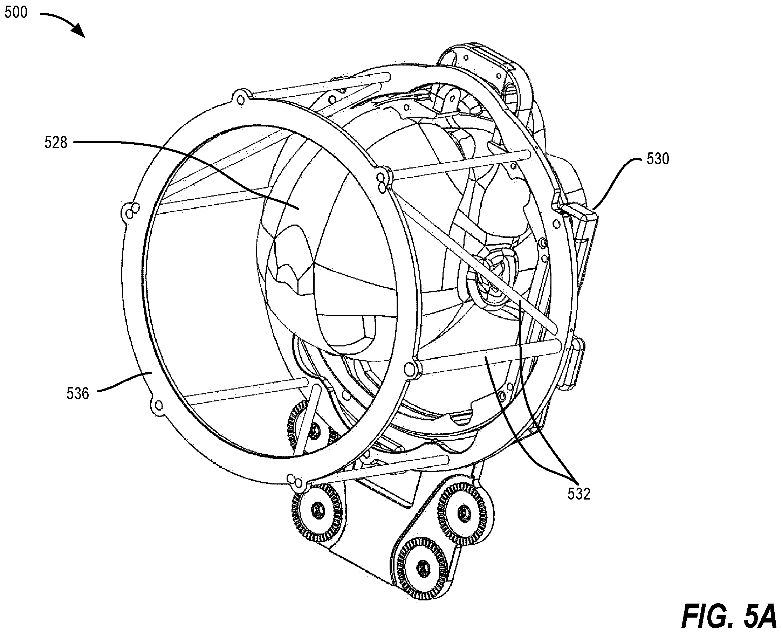

In one example, FIGS. 5A-5C illustrate a system 500 for registering an anatomical feature of a patient. In this example, the stereotactic frame base 530 is fixed to an anatomical feature 528 of patient, e.g., the patient's head. As shown by FIG. 5A, the stereotactic frame base 530 may be affixed to the patient's head 528 prior to registration using pins clamping the skull or other method. The stereotactic frame base 530 may act as both a fixation platform, for holding the patient's head 528 in a fixed position, and registration and tracking platform, for alternatingly holding the CT localizer 536 or the FRA fixture 534. The CT localizer 536 includes a plurality of fiducial markers 532 (e.g., N-pattern radio-opaque rods or other fiducials), which are automatically detected in the image space using image processing. Due to the precise attachment mechanism of the CT localizer 536 to the base 530, these fiducial markers 532 are in known space relative to the stereotactic frame base 530. A 3D CT scan of the patient with CT localizer 536 attached is taken, with an image volume that includes both the patient's head 528 and the fiducial markers 532 of the CT localizer 536. This registration image can be taken intraoperatively or preoperatively, either in the operating room or in radiology, for example. The captured 3D image dataset is stored to computer memory.

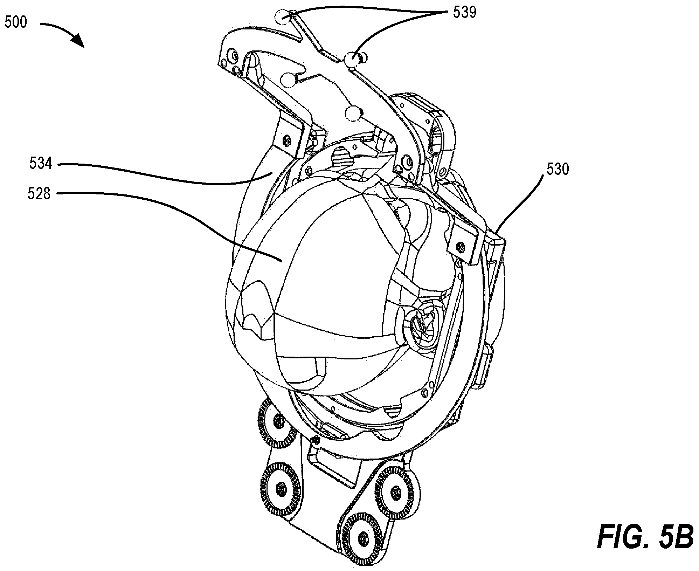

As shown by FIG. 5B, after the registration image is captured, the CT localizer 536 is removed from the stereotactic frame base 530 and the frame reference array fixture 534 is attached to the stereotactic frame base 530. The stereotactic frame base 530 remains fixed to the patient's head 528, however, and is used to secure the patient during surgery, and serves as the attachment point of a frame reference array fixture 534. The frame reference array fixture 534 includes a frame reference array (FRA), which is a rigid array of three or more tracked markers 539, which may be the primary reference for optical tracking. By positioning the tracked markers 539 of the FRA in a fixed, known location and orientation relative to the stereotactic frame base 530, the position and orientation of the patient's head 528 may be tracked in real time. Mount points on the FRA fixture 534 and stereotactic frame base 530 may be designed such that the FRA fixture 534 attaches reproducibly to the stereotactic frame base 530 with minimal (i.e., submillimetric) variability. These mount points on the stereotactic frame base 530 can be the same mount points used by the CT localizer 536, which is removed after the scan has been taken. An auxiliary arm (such as auxiliary arm 107 of FIG. 1B, for example) or other attachment mechanism can also be used to securely affix the patient to the robot base to ensure that the robot base is not allowed to move relative to the patient.

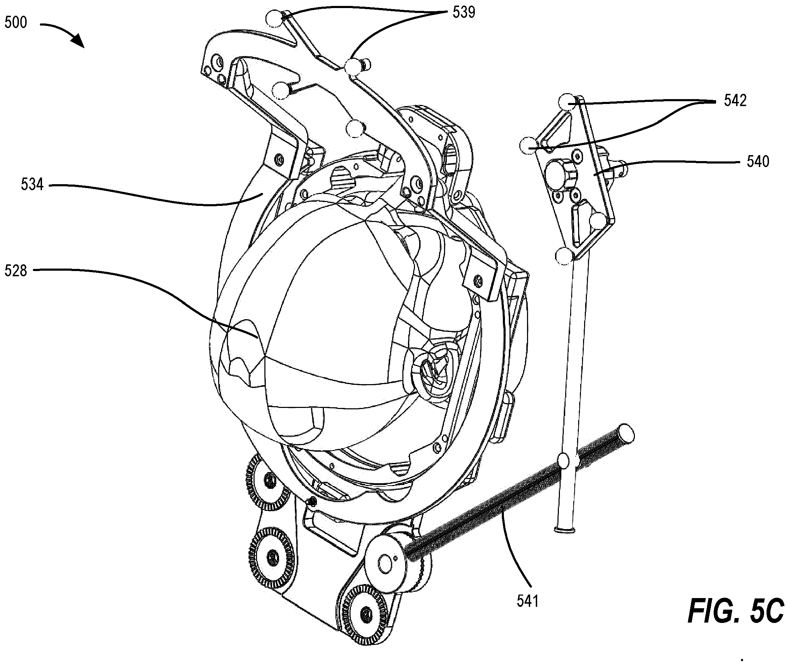

As shown by FIG. 5C, a dynamic reference base (DRB) 540 may also be attached to the stereotactic frame base 530. The DRB 540 in this example includes a rigid array of three or more tracked markers 542. In this example, the DRB 540 and/or other tracked markers may be attached to the stereotactic frame base 530 and/or to directly to the patient's head 528 using auxiliary mounting arms 541, pins, or other attachment mechanisms. Unlike the FRA fixture 534, which mounts in only one way for unambiguous localization of the stereotactic frame base 530, the DRB 540 in general may be attached as needed for allowing unhindered surgical and equipment access. Once the DRB 540 and FRA fixture 534 are attached, registration, which was initially related to the tracking markers 539 of the FRA, can be optionally transferred or related to the tracking markers 542 of the DRB 540. For example, if any part of the FRA fixture 534 blocks surgical access, the surgeon may remove the FRA fixture 534 and navigate using only the DRB 540. However, if the FRA fixture 534 is not in the way of the surgery, the surgeon could opt to navigate from the FRA markers 539, without using a DRB 540, or may navigate using both the FRA markers 539 and the DRB 540. In this example, the FRA fixture 534 and/or DRB 540 uses optical markers, the tracked positions of which are in known locations relative to the stereotactic frame base 530, similar to the CT localizer 536, but it should be understood that many other additional and/or alternative techniques may be used.

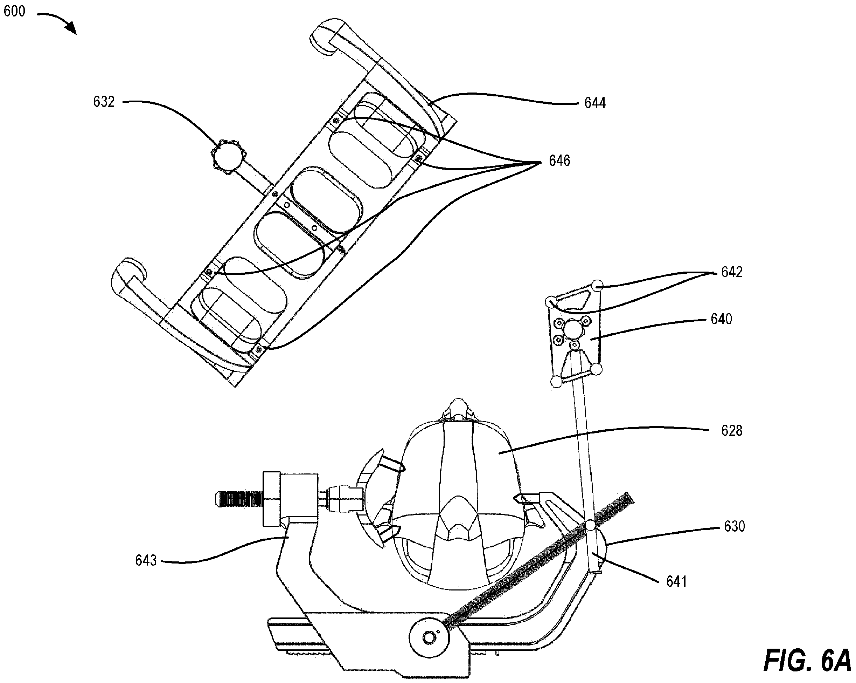

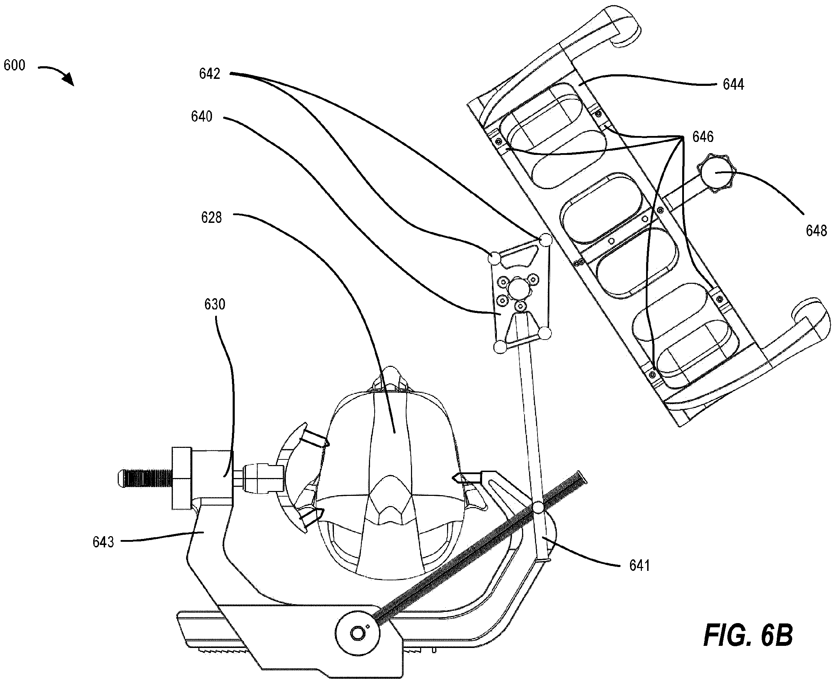

FIGS. 6A and 6B illustrate a system 600 for registering an anatomical feature of a patient using fluoroscopy (fluoro) imaging, according to some embodiments. In this embodiment, image space is registered to tracking space using multiple intraoperative fluoroscopy (fluoro) images taken using a tracked registration fixture 644. The anatomical feature of the patient (e.g., the patient's head 628) is positioned and rigidly affixed in a clamping apparatus 643 in a static position for the remainder of the procedure. The clamping apparatus 643 for rigid patient fixation can be a three-pin fixation system such as a Mayfield clamp, a stereotactic frame base attached to the surgical table, or another fixation method, as desired. The clamping apparatus 643 may also function as a support structure for a patient tracking array or DRB 640 as well. The DRB may be attached to the clamping apparatus using auxiliary mounting arms 641 or other means.

Once the patient is positioned, the fluoro fixture 644 is attached the fluoro unit's x-ray collecting image intensifier (not shown) and secured by tightening clamping feet 632. The fluoro fixture 644 contains fiducial markers (e.g., metal spheres laid out across two planes in this example, not shown) that are visible on 2D fluoro images captured by the fluoro image capture device and can be used to calculate the location of the x-ray source relative to the image intensifier, which is typically about 1 meter away contralateral to the patient, using a standard pinhole camera model. Detection of the metal spheres in the fluoro image captured by the fluoro image capture device also enables the software to de-warp the fluoro image (i.e., to remove pincushion and s-distortion). Additionally, the fluoro fixture 644 contains 3 or more tracking markers 646 for determining the location and orientation of the fluoro fixture 644 in tracking space. In some embodiments, software can project vectors through a CT image volume, based on a previously captured CT image, to generate synthetic images based on contrast levels in the CT image that appear similar to the actual fluoro images (i.e., digitally reconstructed radiographs (DRRs)). By iterating through theoretical positions of the fluoro beam until the DRRs match the actual fluoro shots, a match can be found between fluoro image and DRR in two or more perspectives, and based on this match, the location of the patient's head 628 relative to the x-ray source and detector is calculated. Because the tracking markers 646 on the fluoro fixture 644 track the position of the image intensifier and the position of the x-ray source relative to the image intensifier is calculated from metal fiducials on the fluoro fixture 644 projected on 2D images, the position of the x-ray source and detector in tracking space are known and the system is able to achieve image-to-tracking registration.

As shown by FIG. 6A and 6B, two or more shots are taken of the head 628 of the patient by the fluoro image capture device from two different perspectives while tracking the array markers 642 of the DRB 640, which is fixed to the registration fixture 630 via a mounting arm 641, and tracking markers 646 on the fluoro fixture 644. Based on the tracking data and fluoro data, an algorithm computes the location of the head 628 or other anatomical feature relative to the tracking space for the procedure. Through image-to-tracking registration, the location of any tracked tool in the image volume space can be calculated.

For example, in one embodiment, a first fluoro image taken from a first fluoro perspective can be compared to a first DRR constructed from a first perspective through a CT image volume, and a second fluoro image taken from a second fluoro perspective can be compared to a second DRR constructed from a second perspective through the same CT image volume. Based on the comparisons, it may be determined that the first DRR is substantially equivalent to the first fluoro image with respect to the projected view of the anatomical feature, and that the second DRR is substantially equivalent to the second fluoro image with respect to the projected view of the anatomical feature. Equivalency confirms that the position and orientation of the x-ray path from emitter to collector on the actual fluoro machine as tracked in camera space matches the position and orientation of the x-ray path from emitter to collector as specified when generating the DRRs in CT space, and therefore registration of tracking space to CT space is achieved.

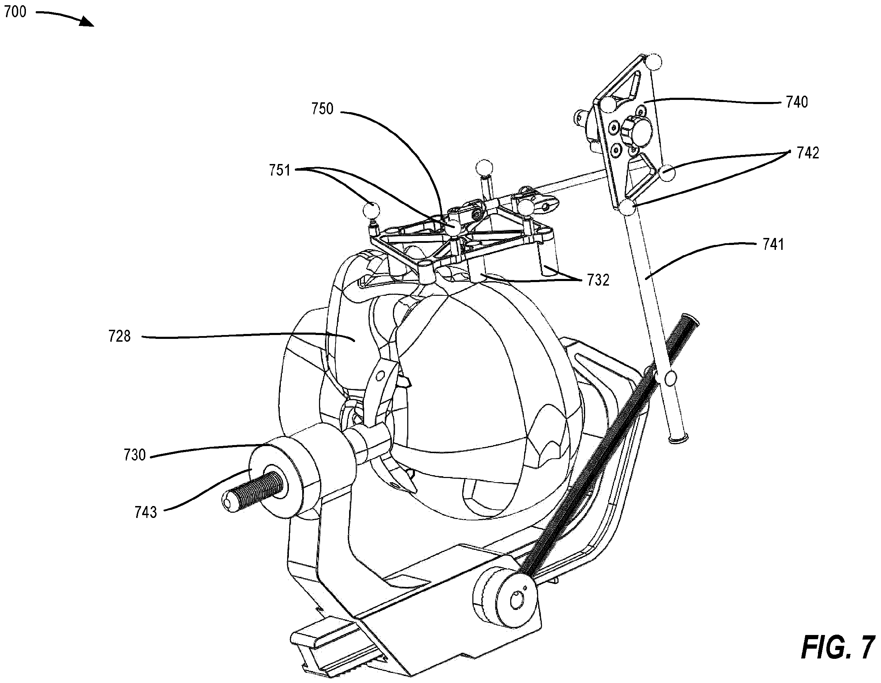

FIG. 7 illustrates a system 700 for registering an anatomical feature of a patient using an intraoperative CT fixture (ICT) and a DRB, according to some embodiments. As shown in FIG. 7, in one application, a fiducial-based image-to-tracking registration can be utilized that uses an intraoperative CT fixture (ICT) 750 having a plurality of tracking markers 751 and radio-opaque fiducial reference markers 732 to register the CT space to the tracking space. After stabilizing the anatomical feature 728 (e.g., the patient's head) using clamping apparatus 730 such as a three-pin Mayfield frame and/or stereotactic frame, the surgeon will affix the ICT 750 to the anatomical feature 728, DRB 740, or clamping apparatus 730, so that it is in a static position relative to the tracking markers 742 of the DRB 740, which may be held in place by mounting arm 741 or other rigid means. A CT scan is captured that encompasses the fiducial reference markers 732 of the ICT 750 while also capturing relevant anatomy of the anatomical feature 728. Once the CT scan is loaded in the software, the system auto-identifies (through image processing) locations of the fiducial reference markers 732 of the ICT within the CT volume, which are in a fixed position relative to the tracking markers of the ICT 750, providing image-to-tracking registration. This registration, which was initially based on the tracking markers 751 of the ICT 750, is then related to or transferred to the tracking markers 742 of the DRB 740, and the ICT 750 may then be removed.

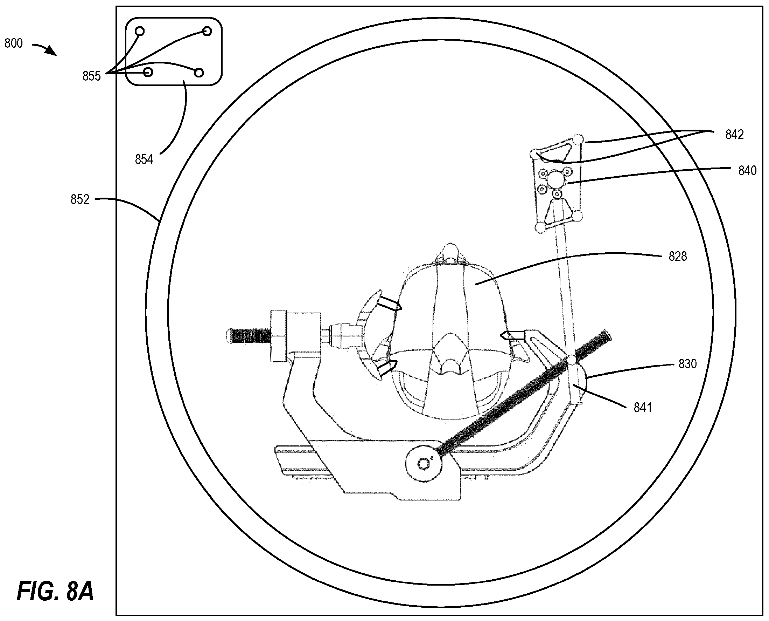

FIG. 8A illustrates a system 800 for registering an anatomical feature of a patient using a DRB and an X-ray cone beam imaging device, according to some embodiments. An intraoperative scanner 852, such as an X-ray machine or other scanning device, may have a tracking array 854 with tracking markers 855, mounted thereon for registration. Based on the fixed, known position of the tracking array 854 on the scanning device, the system may be calibrated to directly map (register) the tracking space to the image space of any scan acquired by the system. Once registration is achieved, the registration, which is initially based on the tracking markers 855 (e.g. gantry markers) of the scanner's array 854, is related or transferred to the tracking markers 842 of a DRB 840, which may be fixed to a clamping fixture 830 holding the patient's head 828 by a mounting arm 841 or other rigid means. After transferring registration, the markers on the scanner are no longer used and can be removed, deactivated or covered if desired. Registering the tracking space to any image acquired by a scanner in this way may avoid the need for fiducials or other reference markers in the image space in some embodiments.

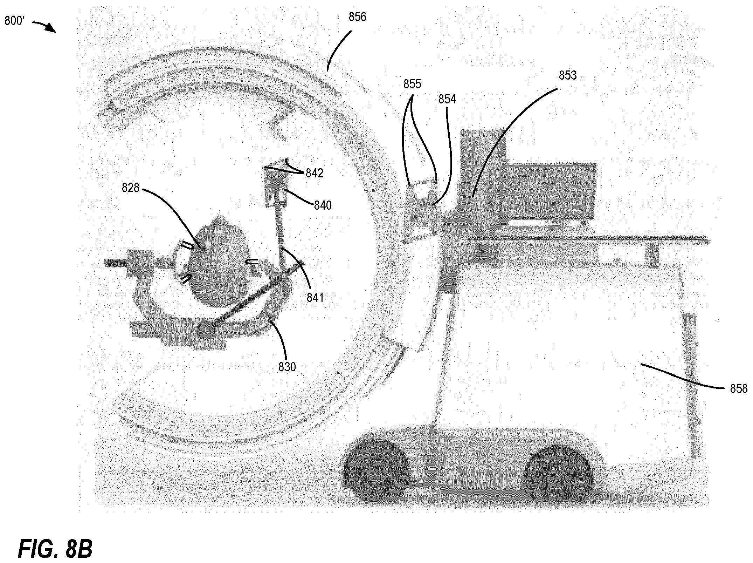

FIG. 8B illustrates an alternative system 800' that uses a portable intraoperative scanner, referred to herein as a C-arm scanner 853. In this example, the C-arm scanner 853 includes a c-shaped arm 856 coupled to a movable base 858 to allow the C-arm scanner 853 to be moved into place and removed as needed, without interfering with other aspects of the surgery. The arm 856 is positioned around the patient's head 828 intraoperatively, and the arm 856 is rotated and/or translated with respect to the patient's head 828 to capture the X-ray or other type of scan that to achieve registration, at which point the C-arm scanner 853 may be removed from the patient.

Another registration method for an anatomical feature of a patient, e.g., a patient's head, may be to use a surface contour map of the anatomical feature, according to some embodiments. A surface contour map may be constructed using a navigated or tracked probe, or other measuring or sensing device, such as a laser pointer, 3D camera, etc. For example, a surgeon may drag or sequentially touch points on the surface of the head with the navigated probe to capture the surface across unique protrusions, such as zygomatic bones, superciliary arches, bridge of nose, eyebrows, etc. The system then compares the resulting surface contours to contours detected from the CT and/or MR images, seeking the location and orientation of contour that provides the closest match. To account for movement of the patient and to ensure that all contour points are taken relative to the same anatomical feature, each contour point is related to tracking markers on a DRB on the patient at the time it is recorded. Since the location of the contour map is known in tracking space from the tracked probe and tracked DRB, tracking-to-image registration is obtained once the corresponding contour is found in image space.

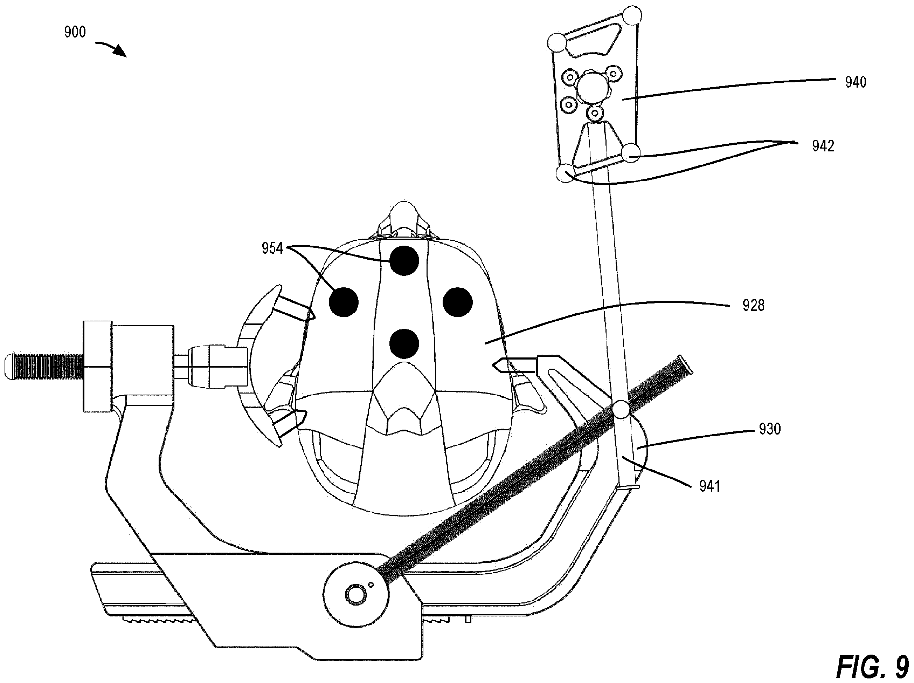

FIG. 9 illustrates a system 900 for registering an anatomical feature of a patient using a navigated or tracked probe and fiducials for point-to-point mapping of the anatomical feature 928 (e.g., a patient's head), according to some embodiments. Software would instruct the user to point with a tracked probe to a series of anatomical landmark points that can be found in the CT or MR image. When the user points to the landmark indicated by software, the system captures a frame of tracking data with the tracked locations of tracking markers on the probe and on the DRB. From the tracked locations of markers on the probe, the coordinates of the tip of the probe are calculated and related to the locations of markers on the DRB. Once 3 or more points are found in both spaces, tracking-to-image registration is achieved. As an alternative to pointing to natural anatomical landmarks, fiducials 954 (i.e., fiducial markers), such as sticker fiducials or metal fiducials, may be used. The surgeon will attach the fiducials 954 to the patient, which are constructed of material that is opaque on imaging, for example containing metal if used with CT or Vitamin E if used with MR. Imaging (CT or MR) will occur after placing the fiducials 954. The surgeon or user will then manually find the coordinates of the fiducials in the image volume, or the software will find them automatically with image processing. After attaching a DRB 940 with tracking markers 942 to the patient through a mounting arm 941 connected to a clamping apparatus 930 or other rigid means, the surgeon or user may also locate the fiducials 954 in physical space relative to the DRB 940 by touching the fiducials 954 with a tracked probe while simultaneously recording tracking markers on the probe (not shown) and on the DRB 940. Registration is achieved because the coordinates of the same points are known in the image space and the tracking space.

One use for the embodiments described herein is to plan trajectories and to control a robot to move into a desired trajectory, after which the surgeon will place implants such as electrodes through a guide tube held by the robot. Additional functionalities include exporting coordinates used with existing stereotactic frames, such as a Leksell frame, which uses five coordinates: X, Y, Z, Ring Angle and Arc Angle. These five coordinates are established using the target and trajectory identified in the planning stage relative to the image space and knowing the position and orientation of the ring and arc relative to the stereotactic frame base or other registration fixture.

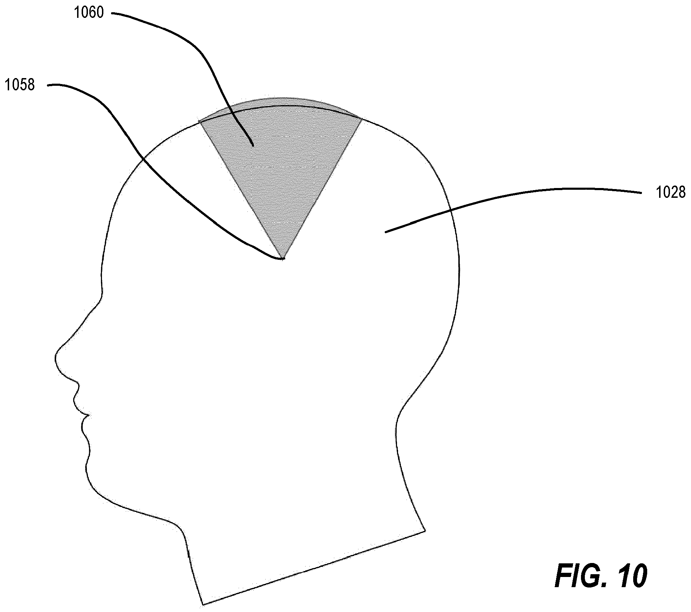

As shown in FIG. 10, stereotactic frames allow a target location 1058 of an anatomical feature 1028 (e.g., a patient's head) to be treated as the center of a sphere and the trajectory can pivot about the target location 1058. The trajectory to the target location 1058 is adjusted by the ring and arc angles of the stereotactic frame (e.g., a Leksell frame). These coordinates may be set manually, and the stereotactic frame may be used as a backup or as a redundant system in case the robot fails or cannot be tracked or registered successfully. The linear x,y,z offsets to the center point (i.e., target location 1058) are adjusted via the mechanisms of the frame. A cone 1060 is centered around the target location 1058, and shows the adjustment zone that can be achieved by modifying the ring and arc angles of the Leksell or other type of frame. This figure illustrates that a stereotactic frame with ring and arc adjustments is well suited for reaching a fixed target location from a range of angles while changing the entry point into the skull.

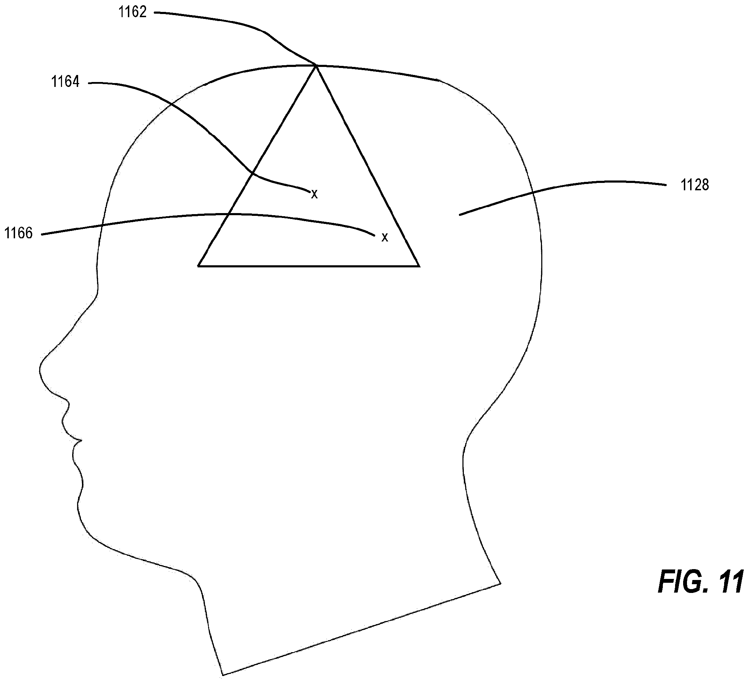

FIG. 11 illustrates a two-dimensional visualization of virtual point rotation mechanism, according to some embodiments. In this embodiment, the robotic arm is able to create a different type of point-rotation functionality that enables a new movement mode that is not easily achievable with a 5-axis mechanical frame, but that may be achieved using the embodiments described herein. Through coordinated control of the robot's axes using the registration techniques described herein, this mode allows the user to pivot the robot's guide tube about any fixed point in space. For example, the robot may pivot about the entry point 1162 into the anatomical feature 1128 (e.g., a patient's head). This entry point pivoting is advantageous as it allows the user to make a smaller burr hole without limiting their ability to adjust the target location 1164 intraoperatively. The cone 1160 represents the range of trajectories that may be reachable through a single entry hole. Additionally, entry point pivoting is advantageous as it allows the user to reach two different target locations 1164 and 1166 through the same small entry burr hole. Alternately, the robot may pivot about a target point (e.g., location 1058 shown in FIG. 10) within the skull to reach the target location from different angles or trajectories, as illustrated in Figure 10. Such interior pivoting robotically has the same advantages as a stereotactic frame as it allows the user to approach the same target location 1058 from multiple approaches, such as when irradiating a tumor or when adjusting a path so that critical structures such as blood vessels or nerves will not be crossed when reaching targets beyond them. Unlike a stereotactic frame, which relies on fixed ring and arc articulations to keep a target/pivot point fixed, the robot adjusts the pivot point through controlled activation of axes and the robot can therefore dynamically adjust its pivot point and switch as needed between the modes illustrated in Figures 10 and 11.

Following the insertion of implants or instrumentation using the robot or ring and arc fixture, these and other embodiments may allow for implant locations to be verified using intraoperative imaging. Placement accuracy of the instrument or implant relative to the planned trajectory can be qualitatively and/or quantitatively shown to the user. One option for comparing planned to placed position is to merge a postoperative verification CT image to any of the preoperative images. Once pre- and post-operative images are merged and plan is shown overlaid, the shadow of the implant on postop CT can be compared to the plan to assess accuracy of placement. Detection of the shadow artifact on post-op CT can be performed automatically through image processing and the offset displayed numerically in terms of millimeters offset at the tip and entry and angular offset along the path. This option does not require any fiducials to be present in the verification image since image-to-image registration is performed based on bony anatomical contours.

A second option for comparing planned position to the final placement would utilize intraoperative fluoro with or without an attached fluoro fixture. Two out-of-plane fluoro images will be taken and these fluoro images will be matched to DRRs generated from pre-operative CT or MR as described above for registration. Unlike some of the registration methods described above, however, it may be less important for the fluoro images to be tracked because the key information is where the electrode is located relative to the anatomy in the fluoro image. The linear or slightly curved shadow of the electrode would be found on a fluoro image, and once the DRR corresponding to that fluoro shot is found, this shadow can be replicated in the CT image volume as a plane or sheet that is oriented in and out of the ray direction of the fluoro image and DRR. That is, the system may not know how deep in or out of the fluoro image plane the electrode lies on a given shot, but can calculate the plane or sheet of possible locations and represent this plane or sheet on the 3D volume. In a second fluoro view, a different plane or sheet can be determined and overlaid on the 3D image. Where these two planes or sheets intersect on the 3D image is the detected path of the electrode. The system can represent this detected path as a graphic on the 3D image volume and allow the user to reslice the image volume to display this path and the planned path from whatever perspective is desired, also allowing automatic or manual calculation of the deviation from planned to placed position of the electrode. Tracking the fluoro fixture is unnecessary but may be done to help de-warp the fluoro images and calculate the location of the x-ray emitter to improve accuracy of DRR calculation, the rate of convergence when iterating to find matching DRR and fluoro shots, and placement of sheets/planes representing the electrode on the 3D scan.

In this and other examples, it is desirable to maintain navigation integrity, i.e., to ensure that the registration and tracking remain accurate throughout the procedure. Two primary methods to establish and maintain navigation integrity include: tracking the position of a surveillance marker relative to the markers on the DRB, and checking landmarks within the images. In the first method, should this position change due to, for example, the DRB being bumped, then the system may alert the user of a possible loss of navigation integrity. In the second method, if a landmark check shows that the anatomy represented in the displayed slices on screen does not match the anatomy at which the tip of the probe points, then the surgeon will also become aware that there is a loss of navigation integrity. In either method, if using the registration method of CT localizer and frame reference array (FRA), the surgeon has the option to re-attach the FRA, which mounts in only one possible way to the frame base, and to restore tracking-to-image registration based on the FRA tracking markers and the stored fiducials from the CT localizer 536. This registration can then be transferred or related to tracking markers on a repositioned DRB. Once registration is transferred the FRA can be removed if desired.

In the above-description of various embodiments of present inventive concepts, it is to be understood that the terminology used herein is for the purpose of describing particular embodiments only and is not intended to be limiting of present inventive concepts. Unless otherwise defined, all terms (including technical and scientific terms) used herein have the same meaning as commonly understood by one of ordinary skill in the art to which present inventive concepts belong. It will be further understood that terms, such as those defined in commonly used dictionaries, should be interpreted as having a meaning that is consistent with their meaning in the context of this specification and the relevant art and will not be interpreted in an idealized or overly formal sense unless expressly so defined herein.

When an element is referred to as being "connected", "coupled", "responsive", or variants thereof to another element, it can be directly connected, coupled, or responsive to the other element or intervening elements may be present. In contrast, when an element is referred to as being "directly connected", "directly coupled", "directly responsive", or variants thereof to another element, there are no intervening elements present. Like numbers refer to like elements throughout. Furthermore, "coupled", "connected", "responsive", or variants thereof as used herein may include wirelessly coupled, connected, or responsive. As used herein, the singular forms "a", "an" and "the" are intended to include the plural forms as well, unless the context clearly indicates otherwise. Well-known functions or constructions may not be described in detail for brevity and/or clarity. The term "and/or" includes any and all combinations of one or more of the associated listed items.

It will be understood that although the terms first, second, third, etc. may be used herein to describe various elements/operations, these elements/operations should not be limited by these terms. These terms are only used to distinguish one element/operation from another element/operation. Thus a first element/operation in some embodiments could be termed a second element/operation in other embodiments without departing from the teachings of present inventive concepts. The same reference numerals or the same reference designators denote the same or similar elements throughout the specification.

As used herein, the terms "comprise", "comprising", "comprises", "include", "including", "includes", "have", "has", "having", or variants thereof are open-ended, and include one or more stated features, integers, elements, steps, components or functions but does not preclude the presence or addition of one or more other features, integers, elements, steps, components, functions or groups thereof. Furthermore, as used herein, the common abbreviation "e.g.", which derives from the Latin phrase "exempli gratia," may be used to introduce or specify a general example or examples of a previously mentioned item, and is not intended to be limiting of such item. The common abbreviation "i.e.", which derives from the Latin phrase "id est," may be used to specify a particular item from a more general recitation.

Example embodiments are described herein with reference to block diagrams and/or flowchart illustrations of computer-implemented methods, apparatus (systems and/or devices) and/or computer program products. It is understood that a block of the block diagrams and/or flowchart illustrations, and combinations of blocks in the block diagrams and/or flowchart illustrations, can be implemented by computer program instructions that are performed by one or more computer circuits. These computer program instructions may be provided to a processor circuit of a general purpose computer circuit, special purpose computer circuit, and/or other programmable data processing circuit to produce a machine, such that the instructions, which execute via the processor of the computer and/or other programmable data processing apparatus, transform and control transistors, values stored in memory locations, and other hardware components within such circuitry to implement the functions/acts specified in the block diagrams and/or flowchart block or blocks, and thereby create means (functionality) and/or structure for implementing the functions/acts specified in the block diagrams and/or flowchart block(s).

These computer program instructions may also be stored in a tangible computer-readable medium that can direct a computer or other programmable data processing apparatus to function in a particular manner, such that the instructions stored in the computer-readable medium produce an article of manufacture including instructions which implement the functions/acts specified in the block diagrams and/or flowchart block or blocks. Accordingly, embodiments of present inventive concepts may be embodied in hardware and/or in software (including firmware, resident software, micro-code, etc.) that runs on a processor such as a digital signal processor, which may collectively be referred to as "circuitry," "a module" or variants thereof.

It should also be noted that in some alternate implementations, the functions/acts noted in the blocks may occur out of the order noted in the flowcharts. For example, two blocks shown in succession may in fact be executed substantially concurrently or the blocks may sometimes be executed in the reverse order, depending upon the functionality/acts involved. Moreover, the functionality of a given block of the flowcharts and/or block diagrams may be separated into multiple blocks and/or the functionality of two or more blocks of the flowcharts and/or block diagrams may be at least partially integrated. Finally, other blocks may be added/inserted between the blocks that are illustrated, and/or blocks/operations may be omitted without departing from the scope of inventive concepts. Moreover, although some of the diagrams include arrows on communication paths to show a primary direction of communication, it is to be understood that communication may occur in the opposite direction to the depicted arrows.

Although several embodiments of inventive concepts have been disclosed in the foregoing specification, it is understood that many modifications and other embodiments of inventive concepts will come to mind to which inventive concepts pertain, having the benefit of teachings presented in the foregoing description and associated drawings. It is thus understood that inventive concepts are not limited to the specific embodiments disclosed hereinabove, and that many modifications and other embodiments are intended to be included within the scope of the appended claims. It is further envisioned that features from one embodiment may be combined or used with the features from a different embodiment(s) described herein. Moreover, although specific terms are employed herein, as well as in the claims which follow, they are used only in a generic and descriptive sense, and not for the purposes of limiting the described inventive concepts, nor the claims which follow. Various features and/or potential advantages of inventive concepts are set forth in the following claims.

## Claims

1. A system (600) comprising:
- a processor circuit; and
- a memory comprising machine-readable instructions configured to cause the processor circuit to:
- provide a medical image volume defining an image space (402), the medical image volume comprising:
- an anatomical feature of a patient (628);
- a registration fixture (644) that is fixed with respect to the anatomical feature of the patient (628); and
- a plurality of fiducial markers (532) that are fixed with respect to the registration fixture (644);
- based on the medical image volume, determine, for each fiducial marker of the plurality of fiducial markers (532), a position of the fiducial marker with respect to the image space;
- determine, based on the determined positions of the plurality of fiducial markers (532), a position and orientation of the registration fixture (644) with respect to the anatomical feature (628);
- provide a tracking data frame defining a tracking space, the tracking data frame comprising positions of a first plurality of tracked markers (646) that are fixed with respect to the registration fixture (644); and
- based on the tracking data frame, determine a position of the anatomical feature (628) with respect to the first plurality of tracked markers (646) in the tracking space, **characterised in that**
- the registration fixture (644) comprises a fixture (644) fixedly attached to an image intensifier of a fluoroscopy (fluoro) image capture device, and
- wherein the medical image volume comprises:
- a first fluoro image of the registration fixture (644), the anatomical feature of the patient (628), and the plurality of fiducial markers that is captured by the fluoro image capture device from a first fluoro image perspective, wherein the plurality of fiducial markers causes a first plurality of shadows on the first fluoro image that are fixed from a first perspective with respect to the registration fixture (644); and
- a second fluoro image of the registration fixture (644), the anatomical feature of the patient (628), and the plurality of fiducial markers that is captured by the fluoro image capture device from a second fluoro image perspective different from the first fluoro image perspective, wherein the plurality of fiducial markers causes a second plurality of shadows on the second fluoro image that are fixed from a second perspective with respect to the registration fixture (644).

2. The system of claim 1, wherein the tracking data frame further comprises positions of a second plurality of tracked markers that are fixed with respect to a patient support structure,
- wherein the anatomical feature of the patient is fixed with respect to the patient support structure, and
- wherein the machine-readable instructions are further configured to cause the processor circuit to:
- based on the tracking data frame and the determined position of the anatomical feature (628) with respect to the first plurality of tracked markers (646) in the tracking space, determine a position of the anatomical feature (628) with respect to the second plurality of tracked markers in the tracking space.

3. The system of claim 2, wherein the first plurality of tracked markers (646) is removed from the patient after determining the position of the anatomical feature (628) with respect to the second plurality of tracked markers (646) in the tracking space.

4. The system of claim 2, wherein the registration fixture comprises a stereotactic frame (530; 730) fixed to the patient.

5. The system of claim 4, wherein the second plurality of tracked markers is fixedly attached to a dynamic reference base (740) (DRB) that is removably attached to the stereotactic frame (530; 730).

6. The system of claim 1, wherein the registration fixture (644) comprises a stereotactic frame fixed to the patient (530; 730).

7. The system of claim 6, wherein the medical image volume comprises a computerized tomography (CT) image of the stereotactic frame (530; 730), the anatomical feature of the patient (628), and a CT localizer that is removably attached to the stereotactic frame (530; 730), the CT localizer comprising the plurality of fiducial markers.

8. The system of claim 6, wherein the first plurality of tracked markers is fixedly attached on a frame reference array (FRA) that is removably attached to the stereotactic frame (530; 730).

9. The system of claim 1, wherein determining the position and orientation of the registration fixture (644) with respect to the anatomical feature further comprises:
- comparing the first fluoro image to a first digitally reconstructed radiograph (DRR) projected through a computerized tomography (CT) image volume having a first DRR perspective;
- determining, based the comparing the first fluoro image to the first DRR, that the first DRR perspective with respect to the anatomical feature is substantially equal to the first fluoro image perspective with respect to the anatomical feature (628);
- comparing the second fluoro image to a second DRR projected through the CT image volume having a second DRR perspective different from the first DRR perspective;
- determining, based comparing the second fluoro image to the DRR, that the second DRR perspective with respect to the anatomical feature (628) is substantially equal to the second fluoro image perspective with respect to the anatomical feature (628);
- determining a position and orientation of the anatomical feature (628) with respect to the first DRR perspective and the second DRR perspective; and
- based on the determining the position and orientation of the anatomical feature (628) with respect to the first DRR perspective and the second DRR perspective, the determining that the first DRR perspective is substantially equal to the first fluoro image perspective, and the determining that the second DRR perspective is substantially equal to the second fluoro image perspective, determining the position and orientation of the registration fixture with respect to the anatomical feature (628).

10. The system of claim 1, wherein the registration fixture (644) comprises an intraoperative computerized tomography (ICT) fixture,
- wherein the plurality of fiducial markers (532) comprise a plurality of radio-opaque fiducial markers that are coupled to the ICT fixture,
- wherein the first plurality of tracked markers (646) are coupled to the ICT fixture,
- wherein the medical image volume comprises a computerized tomography (CT) image of the fiducial markers (532) and the anatomical feature (628) of the patient.

11. The system of claim 1, further comprising:
- an updated medical image volume defining an updated image space comprising:
- the anatomical feature (628) of the patient;
- the registration fixture (644) that is fixed with respect to the anatomical feature of the patient(628); and
- the plurality of fiducial markers (532) that are fixed with respect to the registration fixture (644),
- wherein the machine-readable instructions are further configured to cause the processor circuit to:
- based on the updated medical image volume, determine, for each fiducial marker (532) of the plurality of fiducial markers, an updated position of the fiducial marker (532) with respect to the updated image space;
- determine, based on the determined updated positions of the plurality of fiducial markers (532), an updated position and orientation of the registration fixture with respect to the anatomical feature (628); and
- based on the tracking data frame, determine an updated position of the anatomical feature (628) with respect to the tracking space.

12. The system of claim 1, wherein the tracking data frame further comprises locations of a second plurality of tracked markers that are fixed with respect to a surgical robot (100), and wherein the machine-readable instructions are further configured to cause the processor circuit to:
- based on the tracking data frame, determine a position of a robot arm (641) of the surgical robot (100) with respect to the tracking space;
- determine, based on the determined position and orientation of the anatomical feature (628) with respect to the tracking space and the determined position and orientation of the robot arm (641) with respect to the tracking space, a position and orientation of the anatomical feature (628) with respect to the robot arm (641); and
- control the robot arm (641) based on the determined position and orientation of the anatomical feature (628) with respect to the robot arm (641).

13. The system of claim 12, wherein the machine-readable instructions are further configured to cause the processor circuit to: based on the determined position and orientation of the anatomical feature (628) with respect to the robot arm (641), determine a target location within the anatomical feature (628) in the tracking space; and control the robot arm (641) to position a tool at the target location within the anatomical feature (628).

14. The system of claim 13, wherein the machine-readable instructions are further configured to cause the processor circuit to: based on the determined position and orientation of the anatomical feature (628) with respect to the robot arm (641), determine a pivot location in the tracking space, wherein controlling the robot arm (641) to position the tool at the target location comprises pivoting the tool about the pivot location.

## Patentansprüche

1. System (600), umfassend:
- eine Prozessorschaltung; und
- einen Speicher, der maschinenlesbare Befehle umfasst, die so konfiguriert sind, dass die Prozessorschaltung Folgendes veranlasst:
- Bereitstellen eines medizinischen Bildvolumens, das einen Bildraum (402) definiert, wobei das medizinische Bildvolumen umfasst:
- ein anatomisches Merkmal eines Patienten (628);
- eine Erfassungsvorrichtung (644), die in Bezug auf das anatomische Merkmal des Patienten (628) fixiert ist; und
- eine Vielzahl von Referenzmarkern (532), die in Bezug auf die Erfassungsvorrichtung (644) fixiert sind;
- Ermitteln, basierend auf dem medizinischen Bildvolumen, einer Position der Referenzmarker in Bezug auf den Bildraum für jeden Referenzmarker der Vielzahl von Referenzmarker (532);
- Ermitteln, basierend auf den ermittelten Positionen der Vielzahl von Referenzmarker (532), einer Position und Ausrichtung der Erfassungsvorrichtung (644) in Bezug auf das anatomische Merkmal (628);
- Bereitstellen eines Tracking-Datenrahmens, der einen Tracking-Raum definiert, wobei der Tracking-Datenrahmen Positionen einer ersten Vielzahl von Tracked-Markern (646) umfasst, die in Bezug auf die Erfassungsvorrichtung (644) fixiert sind; und
- Ermitteln einer Position des anatomischen Merkmals (628) in Bezug auf die erste Vielzahl von Tracked-Markern (646) im Tracking-Raum basierend auf dem Tracking-Datenrahmen, **dadurch gekennzeichnet, dass**
- die Erfassungsvorrichtung (644) eine Vorrichtung (644) umfasst, die an einem Bildverstärker einer Fluoroskopie- (Fluoro-) Bildaufnahmevorrichtung fixiert ist, und
- wobei das medizinische Bildvolumen umfasst:
- ein erstes Fluoro-Bild der Erfassungsvorrichtung (644), das anatomische Merkmal des Patienten (628) und die Vielzahl von Referenzmarkern, die von der Fluoro-Bildaufnahmevorrichtung aus einer ersten Fluoro-Bildperspektive erfasst wird, wobei die Vielzahl von Referenzmarkern eine erste Vielzahl von Schatten auf dem ersten Fluoro-Bild verursacht, die aus einer ersten Perspektive in Bezug auf die Erfassungsvorrichtung (644) fixiert sind; und
- ein zweites Fluoro-Bild der Erfassungsvorrichtung (644), das anatomische Merkmal des Patienten (628) und die Vielzahl von Referenzmarkern, das von der Fluoro-Bildaufnahmevorrichtung aus einer zweiten Fluoro-Bildperspektive erfasst wird, die sich von der ersten Fluoro-Bildperspektive unterscheidet, wobei die Vielzahl von Referenzmarkern eine zweite Vielzahl von Schatten auf dem zweiten Fluoro-Bild verursacht, die aus einer zweiten Perspektive in Bezug auf die Erfassungsvorrichtung (644) fixiert sind.

2. System nach Anspruch 1, wobei der Tracking-Datenrahmen ferner Positionen einer zweiten Vielzahl von Tracked-Markern umfasst, die in Bezug auf eine Patientenstützstruktur fixiert sind,
- wobei das anatomische Merkmal des Patienten in Bezug auf die Patientenstützstruktur fixiert ist, und
- wobei die maschinenlesbaren Befehle ferner so konfiguriert sind, dass die Prozessorschaltung Folgendes veranlasst:
- Ermitteln einer Position des anatomischen Merkmals (628) in Bezug auf die zweite Vielzahl von Tracked-Markern im Tracking-Raum basierend auf dem Tracking-Datenrahmen und der ermittelten Position des anatomischen Merkmals (628) in Bezug auf die erste Vielzahl von Tracked-Markern (646) im Tracking-Raum.

3. System nach Anspruch 2, wobei die erste Vielzahl von Tracked-Markern (646) vom Patienten entfernt wird, nachdem die Position des anatomischen Merkmals (628) in Bezug auf die zweite Vielzahl von Tracked-Markern (646) im Tracking-Raum ermittelt wurde.

4. System nach Anspruch 2, wobei die Erfassungsvorrichtung einen stereotaktischen Rahmen (530; 730) umfasst, der am Patienten fixiert ist.

5. System nach Anspruch 4, wobei die zweite Vielzahl von Tracked-Markern an einer dynamischen Referenzbasis (740) (DRB) stationär befestigt ist, die am stereotaktischen Rahmen (530; 730) abnehmbar befestigt ist.

6. System nach Anspruch 1, wobei die Erfassungsvorrichtung (644) einen stereotaktischen Rahmen umfasst, der am Patienten (530; 730) befestigt ist.

7. System nach Anspruch 6, wobei das medizinische Bildvolumen ein Computertomographie- (CT-) Bild des stereotaktischen Rahmens (530; 730), das anatomische Merkmal des Patienten (628), und einen CT-Lokalisierer umfasst, der am stereotaktischen Rahmen (530; 730) abnehmbar befestigt ist, wobei der CT-Lokalisierer die Vielzahl von Referenzmarkern umfasst.

8. System nach Anspruch 6, wobei die erste Vielzahl von Tracked-Markern an einem Frame-Referenz-Array (FRA) stationär befestigt ist, das am stereotaktischen Rahmen (530; 730) abnehmbar befestigt ist.

9. System nach Anspruch 1, wobei das Ermitteln der Position und der Ausrichtung der Erfassungsvorrichtung (644) in Bezug auf das anatomische Merkmal ferner umfasst:
- Vergleichen des ersten Fluoro-Bildes mit einem ersten digital rekonstruierten Röntgenbild (DRR), das durch ein Computertomographie- (CT-) Bildvolumen mit einer ersten DRR-Perspektive projiziert wird;
- Ermitteln, basierend auf dem Vergleich des ersten Fluoro-Bildes mit dem ersten DRR, dass die erste DRR-Perspektive in Bezug auf das anatomische Merkmal im Wesentlichen der Perspektive des ersten Fluoro-Bildes in Bezug auf das anatomische Merkmal (628) gleicht;
- Vergleichen des zweiten Fluoro-Bildes mit einem zweiten DRR, das durch das CT-Bildvolumen mit einer zweiten DRR-Perspektive projiziert wird, die sich von der ersten DRR-Perspektive unterscheidet;
- Ermitteln, basierend auf dem Vergleich des zweiten Fluoro-Bildes mit dem DRR, dass die zweite DRR-Perspektive in Bezug auf das anatomische Merkmal (628) im Wesentlichen der zweiten Fluoro-Bildperspektive in Bezug auf das anatomische Merkmal (628 gleicht;
- Ermitteln einer Position und Ausrichtung des anatomischen Merkmals (628) in Bezug auf die erste DRR-Perspektive und die zweite DRR-Perspektive; und
- basierend auf dem Ermitteln der Position und Ausrichtung des anatomischen Merkmals (628) in Bezug auf die erste DRR-Perspektive und die zweite DRR-Perspektive, dem Ermitteln, dass die erste DRR-Perspektive im Wesentlichen der ersten Fluoro-Bildperspektive gleicht, und dem Ermitteln, dass die zweite DRR-Perspektive im Wesentlichen der zweiten Fluoro-Bildperspektive gleicht, Ermitteln der Position und Ausrichtung der Erfassungsvorrichtung in Bezug auf das anatomische Merkmal (628).

10. System nach Anspruch 1, wobei die Erfassungsvorrichtung (644) eine intraoperative Computertomographievorrichtung (ICT) umfasst,
- wobei die Vielzahl von Referenzmarkern (532) eine Vielzahl von strahlenundurchlässigen Referenzmarkern umfasst, die mit der ICT-Vorrichtung verbunden sind,
- wobei die erste Vielzahl von Tracked-Markern (646) mit der ICT-Vorrichtung verbunden ist,
- wobei das medizinische Bildvolumen ein Computertomographie- (CT-) Bild der Referenzmarker (532) und des anatomischen Merkmals (628) des Patienten umfasst.

11. System nach Anspruch 1, ferner umfassend:
- ein aktualisiertes medizinisches Bildvolumen, das einen aktualisierten Bildraum definiert, umfassend:
- das anatomische Merkmal (628) des Patienten;
- die Erfassungsvorrichtung (644), die in Bezug auf die anatomischen Merkmale des Patienten (628) fixiert ist; und
- die Vielzahl von Referenzmarkern (532), die in Bezug auf die Erfassungsvorrichtung (644) fixiert sind,
- wobei die maschinenlesbaren Befehle ferner so konfiguriert sind, dass die Prozessorschaltung Folgendes veranlasst:
- Ermitteln, basierend auf dem aktualisierten medizinischen Bildvolumen, einer aktualisierten Position des Referenzmarkers (532) in Bezug auf den aktualisierten Bildraum für jeden Referenzmarker (532) der Vielzahl von Referenzmarkern;
- Ermitteln, basierend auf den ermittelten aktualisierten Positionen der Vielzahl von Referenzmarkern (532), einer aktualisierten Position und Ausrichtung der Registrierungshalterung in Bezug auf das anatomische Merkmal (628); und
- Ermitteln, basierend auf dem Tracking-Datenrahmen, einer aktualisierten Position des anatomischen Merkmals (628) in Bezug auf den Tracking-Raum.

12. System nach Anspruch 1, wobei der Tracking-Datenrahmen ferner Positionen einer zweiten Vielzahl von Tracked-Markern umfasst, die in Bezug auf einen chirurgischen Roboter (100) fixiert sind, und wobei die maschinenlesbaren Befehle ferner so konfiguriert sind, dass die Prozessorschaltung Folgendes veranlasst:
- Ermitteln, basierend auf dem Tracking-Datenrahmen, einer Position eines Roboterarms (641) des chirurgischen Roboters (100) in Bezug auf den Tracking-Raum;
- Ermitteln, basierend auf der ermittelten Position und Ausrichtung des anatomischen Merkmals (628) in Bezug auf den Tracking-Raum und der ermittelten Position und Ausrichtung des Roboterarms (641) in Bezug auf den Tracking-Raum, einer Position und Ausrichtung des anatomischen Merkmals (628) in Bezug auf den Roboterarm (641); und
- Steuern des Roboterarms (641) basierend auf der ermittelten Position und Ausrichtung des anatomischen Merkmals (628) in Bezug auf den Roboterarm (641).

13. System nach Anspruch 12, wobei die maschinenlesbaren Befehle ferner so konfiguriert sind, dass die Prozessorschaltung Folgendes veranlasst: Ermitteln, basierend auf der ermittelten Position und Ausrichtung des anatomischen Merkmals (628) in Bezug auf den Roboterarm (641), einer Zielposition in Innern des anatomischen Merkmals (628) im Tracking-Raum; und Steuern des Roboterarms (641), um ein Werkzeug an der Zielposition im Innern des anatomischen Merkmals (628) zu positionieren.

14. System nach Anspruch 13, wobei die maschinenlesbaren Befehle ferner so konfiguriert sind, dass die Prozessorschaltung Folgendes veranlasst: Ermitteln, basierend auf der ermittelten Position und Ausrichtung des anatomischen Merkmals (628) in Bezug auf den Roboterarm (641), einer Schwenkposition im Tracking-Raum, wobei das Steuern des Roboterarms (641) zum Positionieren des Werkzeugs an der Zielposition das Schwenken des Werkzeugs um die Schwenkposition umfasst.

## Revendications

1. Système (600) comprenant :
- un circuit de processeur ; et
- une mémoire comprenant des instructions lisibles par machine configurées pour amener le circuit de processeur à :
- fournir un volume d'image médicale définissant un espace d'image (402), le volume d'image médicale comprenant :
- une caractéristique anatomique d'un patient (628) ;
- un appareil d'enregistrement (644) qui est fixe par rapport à la caractéristique anatomique du patient (628) ; et
- une pluralité de marqueurs de calage (532) qui sont fixes par rapport à l'appareil d'enregistrement (644) ;
- sur la base du volume d'image médicale, déterminer, pour chaque marqueur de calage de la pluralité de marqueurs de calage (532), une position du marqueur de calage par rapport à l'espace d'image ;
- déterminer, sur la base des positions déterminées de la pluralité de marqueurs de calage (532), une position et une orientation de l'appareil d'enregistrement (644) par rapport à la caractéristique anatomique (628) ;
- fournir un cadre de données de suivi définissant un espace de suivi, le cadre de données de suivi comprenant des positions d'une première pluralité de marqueurs suivis (646) qui sont fixes par rapport à l'appareil d'enregistrement (644) ; et
- sur la base du cadre de données de suivi, déterminer une position de la caractéristique anatomique (628) par rapport à la première pluralité de marqueurs suivis (646) dans l'espace de suivi, **caractérisé en ce que**
- l'appareil d'enregistrement (644) comprend un appareil (644) fermement fixé à un intensificateur d'images d'un dispositif de capture d'image fluoroscopique (fluoro), et
- dans lequel le volume d'image médicale comprend :
- une première image fluoro de l'appareil d'enregistrement (644), la caractéristique anatomique du patient (628), et la pluralité de marqueurs de calage qui est capturée par le dispositif de capture d'image fluoro d'une première perspective d'image fluoro, dans lequel la pluralité de marqueurs de calage entraîne une première pluralité d'ombres sur la première image fluoro qui sont fixes d'une première perspective par rapport à l'appareil d'enregistrement (644) ; et
- une seconde image fluoro de l'appareil d'enregistrement (644), la caractéristique anatomique du patient (628), et la pluralité de marqueurs de calage qui est capturée par le dispositif de capture d'image fluoro d'une seconde perspective d'image fluoro différente de la première perspective d'images fluoro, dans lequel la pluralité de marqueurs de calage entraîne une seconde pluralité d'ombres sur la seconde image fluoro qui sont fixes d'une seconde perspective par rapport à l'élément d'enregistrement (644).

2. Système selon la revendication 1, dans lequel le cadre de données de suivi comprend en outre des positions d'une seconde pluralité de marqueurs suivis qui sont fixes par rapport à une structure de support de patient,
- dans lequel la caractéristique anatomique du patient est fixe par rapport à la structure de support de patient, et
- dans lequel les instructions lisibles par machine sont en outre configurées pour amener le circuit de processeur à :
- sur la base du cadre de données de suivi et de la position déterminée de la caractéristique atomique (628) par rapport à la première pluralité de marqueurs suivis (646) dans l'espace de suivi, déterminer une position d'une caractéristique anatomique (628) par rapport à la seconde pluralité de marqueurs suivis dans l'espace de suivi.

3. Système selon la revendication 2, dans lequel la première pluralité de marqueurs suivis (646) est retirée du patient après détermination de la position de la caractéristique anatomique (628) par rapport à la seconde pluralité de marqueurs suivis (646) dans l'espace de suivi.

4. Système selon la revendication 2, dans lequel l'appareil d'enregistrement comprend un cadre stéréotaxique (530 ; 730) fixé au patient.

5. Système selon la revendication 4, dans lequel la seconde pluralité de marqueurs suivis est fermement fixée à une base de référence dynamique (740) (DRB) qui est fixée de manière amovible au cadre stéréotaxique (530 ; 730).

6. Système selon la revendication 1, dans lequel l'appareil d'enregistrement (644) comprend un cadre stéréotaxique fixé au patient (530 ; 730).

7. Système selon la revendication 6, dans lequel le volume d'image médicale comprend une image de tomodensitométrie (TDM) du cadre stéréotaxique (530 ; 730), la caractéristique anatomique du patient (628), et un localisateur de TDM qui est fixé de manière amovible au cadre stéréotaxique (530 ; 730), le localisateur de TDM comprenant la pluralité de marqueurs de calage.

8. Système selon la revendication 6, dans lequel la première pluralité de marqueurs suivis est fermement fixée sur un réseau de référence de cadre (FRA) qui est fixé de manière amovible au cadre stéréotaxique (530 ; 730).

9. Système selon la revendication 1, dans lequel la détermination de la position et de l'orientation de l'appareil d'enregistrement (644) par rapport à la caractéristique anatomique comprend en outre :
- la comparaison de la première image fluoro à une première radiographie reconstruite numériquement (DRR) projetée à travers un volume d'image de tomodensitométrie (TDM) ayant une première perspective de DRR ;
- la détermination, sur la base de la comparaison de la première image fluoro à la première DRR, que la première perspective de DRR par rapport à la caractéristique anatomique est sensiblement égale à la première perspective d'image fluoro par rapport à la caractéristique anatomique (628) ;
- la comparaison de la seconde image fluoro à une seconde DRR projetée à travers le volume d'image de TDM ayant une seconde perspective de DRR différente de la première perspective de DRR ;
- la détermination, sur la base de la comparaison de la seconde image fluoro à la DRR, que la seconde perspective de DRR par rapport à la caractéristique anatomique (628) est sensiblement égale à la seconde perspective d'image fluoro par rapport à la caractéristique anatomique (628) ;
- la détermination d'une position et d'une orientation de la caractéristique anatomique (628) par rapport à la première perspective de DRR et à la seconde perspective de DRR ; et
- sur la base de la détermination de la position et de l'orientation de la caractéristique anatomique (628) par rapport à la première perspective de DRR et à la seconde perspective de DRR, la détermination que la première perspective de DRR est sensiblement égale à la première perspective d'image fluoro, et la détermination que la seconde perspective de DRR est sensiblement égale à la seconde perspective d'image fluoro, la détermination de la position et de l'orientation de l'appareil d'enregistrement par rapport à la caractéristique anatomique (628).

10. Système selon la revendication 1, dans lequel l'appareil d'enregistrement (644) comprend un appareil de tomodensitométrie intra-opératoire (TDMI),
- dans lequel la pluralité de marqueurs de calage (532) comprend une pluralité de marqueurs de calage radio-opaques qui sont couplés à l'appareil de TDMI,
- dans lequel la première pluralité de marqueurs suivis (646) sont couplés à l'appareil de TDMI,
- dans lequel le volume d'image médicale comprend une image de tomodensitométrie (TDM) des marqueurs de calage (532) et de la caractéristique anatomique (628) du patient.

11. Selon la revendication 1, comprenant en outre :
- un volume d'images médicales à jour définissant un espace d'image médicale à jour comprenant :
- la caractéristique anatomique (628) du patient ;
- l'appareil d'enregistrement (644) qui est fixe par rapport à la caractéristique anatomique du patient (628) ; et
- la pluralité de marqueurs de calage (532) qui sont fixes par rapport à l'appareil d'enregistrement (644),
- dans lequel les instructions lisibles par machine sont en outre configurées pour amener le circuit de processeur à :
- sur la base du volume d'image médicale à jour, déterminer, pour chaque marqueur de calage (532) de la pluralité des marqueurs de calage, une position à jour du marqueur de calage (532) par rapport à l'espace d'image à jour ;
- déterminer, sur la base des positions à jour déterminées de la pluralité de marqueurs de calage (532), une position et une orientation à jour de l'appareil d'enregistrement par rapport à la caractéristique anatomique (628) ; et
- sur la base du cadre de données de suivi, déterminer une position à jour de la caractéristique anatomique (628) par rapport à l'espace de suivi.

12. Système selon la revendication 1, dans lequel le cadre de données de suivi comprend en outre des emplacements d'une seconde pluralité de marqueurs suivis qui sont fixes par rapport à un robot chirurgical (100), et dans lequel les instructions lisibles par machine sont en outre configurées pour amener le circuit de processeur à :
- sur la base du cadre de données de suivi, déterminer une position d'un bras de robot (641) du robot chirurgical (100) par rapport à l'espace de suivi ;
- déterminer, sur la base de la position et de l'orientation déterminées de la caractéristique anatomique (628) par rapport à l'espace de suivi et de la position et de l'orientation déterminées du bras de robot (641) par rapport à l'espace de suivi, une position et une orientation de la caractéristique anatomique (628) par rapport au bras de robot (641) ; et
- commander le bras de robot (641) sur la base de la position et de l'orientation déterminées de la caractéristique anatomique (628) par rapport au bras de robot (641).

13. Système selon la revendication 12, dans lequel les instructions lisibles par machine sont en outre configurées pour amener le circuit de processeur à : sur la base de la position et de l'orientation déterminées de la caractéristique anatomique (628) par rapport au bras de robot (641), déterminer un emplacement cible à l'intérieur de la caractéristique anatomique (628) dans l'espace de suivi ; et commander au bras de robot (641) de positionner un outil au niveau de l'emplacement cible à l'intérieur de la caractéristique anatomique (628).

14. Système selon la revendication 13, dans lequel les instructions lisibles par machine sont en outre configurées pour amener le circuit de processeur à : sur la base de la position et de l'orientation déterminées de la caractéristique anatomique (628) par rapport au bras de robot (641), déterminer un emplacement pivot de l'espace de suivi, dans lequel la commande du bras de robot (641) pour positionner l'outil au niveau de l'emplacement cible comprend le fait de faire pivoter l'outil autour de l'emplacement pivot.
